# EUROPÄISCHE PATENTSCHRIFT

(11) **EP 0 679 627 B1**
(45) Veröffentlichungstag und Bekanntmachung des Hinweises auf die Patenterteilung: **09.09.1998**
(21) Anmeldenummer: 95810260.0
(22) Anmeldetag: 19.04.1995
(51) Int. Cl.: C07C 43/12, C07C 41/28

(54) **Ein Verfahren zur Herstellung von halogenierten Ethern**
Process for the preparation of halogenated ethers
Procédé pour la préparation d'éthers halogénés

(30) Priorität: 28.04.1994 CH 1319/94; 01.11.1994 CH 3265/94
(43) Veröffentlichungstag der Anmeldung: 02.11.1995
(73) Patentinhaber: Säurefabrik Schweizerhall, CH-4133 Pratteln 4 (CH)
(72) Erfinder: Gallegra, Pasquale, Dr., CH-4132 Muttenz (CH); Degischer, Gerhard, Dr., CH-4414 Füllinsdorf (CH)
(74) Vertreter: Schluep, Hans-Peter

(56) Entgegenhaltungen:
- DE-A- 2 431 778
- DE-C- 880 285
- US-A- 3 972 947
- CHEMICAL ABSTRACTS, vol. 89, no. 15, 9. Oktober 1978, Columbus, Ohio, US; abstract no. 129340c, V. A. TOPCHII ET AL 'Synthesis and cyclization of beta-substituted alpha-chloro ethers' Seite 574 ;Spalte 2 ;
- CHEMICAL ABSTRACTS, vol. 95, no. 17, 26. Oktober 1981, Columbus, Ohio, US; abstract no. 149932z, 'beta-Alkoxyethoxymethyl halides' Seite 600 ;Spalte 1 ;
- MACROMOLECULES, Bd.24, Nr.21, 14. Oktober 1991, EASTON US Seiten 5879 - 5880 M. E. WRIGHT ET AL 'Details concerning the chloromethylation of soluble high molecular weight polystyrene using dimethoxymethane, thionyl chloride and a Lewis acid: a full analysis'
- TETRAHEDRON LETTERS., Nr.11, 1976, OXFORD GB Seiten 809 - 812 E. J. COREY ET AL 'A new general method for protection of the hydroxyl function'

## Beschreibung

Die Erfindung betrifft ein neuartiges Verfahren zur Herstellung von 1-halogenierten Ethern, welche bei der Herstellung von biologisch wirksamen Verbindungen, z.B. Pharmazeutika oder Fungiziden, Verwendung finden.

Das erfindungsgemässe Verfahren zur Herstellung von Verbindungen der Formel I, 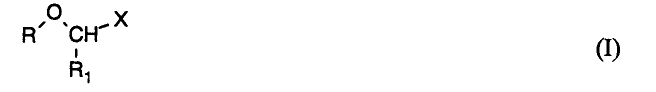
worin R ein- oder zweifach substituiertes Niederalkyl bedeutet, worin die Substituenten aus Halogen und Niederalkoxy ausgewählt sind, mit der Massgabe, dass diese Substituenten nicht am die Gruppe R an den Rest des Moleküls der Formel I bindenden Kohlenstoffatom des Niederalkylrestes R vorliegen;
R₁ Wasserstoff, Niederalkyl, Phenyl oder Phenylniederalkyl bedeutet; und
X Chlor oder Brom bedeutet;
   ist dadurch gekennzeichnet, dass man ein Acetal der Formel II, 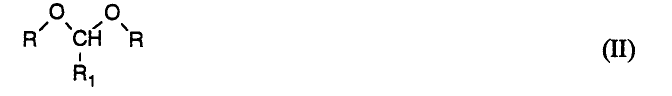 worin R und R₁ die oben genannten Bedeutungen haben,
   mit mindestens einer Verbindung der Formel R₂-X, worin R₂ Wasserstoff oder X-SO bedeutet, wobei im letzteren Falle im Reaktionsgemisch noch eine katalytisch wirksame Menge an N,N-Diniederalkyl-niederalkanoylamid(en) vorliegen muss, und worin X die für Verbindungen der Formel I genannten Bedeutungen hat, umsetzt.

Bekannt ist die Herstellung von Verbindungen der Formel I durch Umsetzung der entsprechenden Alkohole der Formel Rₙ-OH, worin Rₙ beispielsweise die unter Formel I für R genannten Bedeutungen hat, mit Formaldehyd oder Paraformaldehyd in Gegenwart von wässrigen Säuren, z.B. der entsprechenden Halogenwasserstoffsäure (HBr oder HCl) (vgl. Olah, G. A., et al., "Haloalkylations", in: Friedel-Crafts and Related Reactions, Vol. II, J. Wiley & Sons, New York, 1964, P. 659-671 and 734-737; und US 4,568,700, published Feb. 4, 1986). Nachteilig an den bekannten Verfahren ist, dass (insbesondere, wenn R in Rₙ-OH ein sekundärer Kohlenwasserstoffrest ist, aber auch, wenn R ein primärer Kohlenwasserstoffrest ist) eine Fülle von vorwiegend unerwünschten Nebenprodukten entstehen, die zur Ausbeuteverminderung führen, darunter insbesondere auch die entsprechenden cancerogenen und somit chronisch toxischen Bis-(1-halogen-niederalk-1-yl)ether, wie beispielsweise Bis(chlormethyl)ether (vgl. Tou., J.C., et al., "Possible Formation of Bis(chloromethyl)ether from the Reactions of Formaldehyde and Chloride Ion", Anal. Chem. 48(7), 958-63 (1976)), und/oder die Aufarbeitung erschweren. DE-OS 243 1778 beschreibt ein Verfahren zur Herstellung von Chlormethyl-methylether durch Lösungsmittelextraktion, wobei diese Extraktion als erfindungswesentlich dargestellt wird, während Destillation zur Reinigung des Produkts als ungeeignet beschrieben wird, im Gegensatz zur hier vorliegenden Erfindung. Das DE-Patent Nr. 880 285 nennt ein Verfahren zur Herstellung von α-Chlorethern, welches von einem Aldehyd, einem Alkohol und SOCl₂ ausgeht. Das nun vorgestellte Verfahren mit SOCl₂ hingegen, welches in Gegenwart eines N,N-Diniederalkyl-niederalkanoylamids abläuft, zeigt jedoch wesentlich bessere Reinheit des Produkts.

Aufgabe der vorliegenden Erfindung ist es, ein neues, vorteilhaftes und wirtschaftliches Verfahren zur Herstellung von Verbindungen der Formel I bereitzustellen, welches auch die genannten Nachteile vermeidet und insbesondere das Erzielen hoher Ausbeuten, einer geringen Menge von unerwünschten, nicht weiterverwendbaren und/oder toxischen Nebenprodukten, insbesondere von cancerogenen Nebenprodukten, eines hochreinen Endproduktes und/oder eines Reaktionsverlaufes unter Erhalten von nützlichen, weiterverwendbaren Nebenprodukten ermöglicht und/oder eine einfache Aufarbeitung des erhaltenen Endproduktes ermöglicht.

Dies wird durch das oben genannte und unten weiter im Detail beschriebene Verfahren erreicht.

Niederalkyl ist z.B. geradkettiges oder verzweigtkettiges C₁-C₇-Alkyl, wie Methyl, Ethyl, Propyl, Isopropyl, Butyl, Isobutyl, sec-Butyl, oder ferner Pentyl, Isopentyl, Neopentyl, Hexyl oder Heptyl.

Substituiertes Niederalkyl ist einer der für Niederalkyl genannten Reste, welcher durch einen oder zwei Substituenten ausgewählt aus Halogen, wie Fluor, Iod oder insbesondere Chlor oder Brom, und Niederalkoxy, z.B. Ethoxy oder insbesondere Methoxy, substituiert ist. Bevorzugt ist durch einen oder zwei Substituenten ausgewählt aus Chlor, Brom und Niederalkoxy, wie Methoxy, substituiertes Niederalkyl, wie 1,3-Dichlor- oder 1,3-Dibrom-2-propyl, 2-Chlor- oder 2-Bromethyl, oder 2-Methoxyethyl. Die Substituenten Halogen und/oder Niederalkoxy liegen dabei nicht am bindenden Kohlenstoffatom vor, d. h. an dem Kohlenstoffatom, welches die Gruppe R in Formel I an das mit dem Rest des Moleküls in Formel I verknüpfende Sauerstoffatom bindet.

Sofern nichts anderes angegeben ist, bedeutet der bei der Definition von Resten wie Niederalkyl oder Niederalkoxy verwendete Ausdruck "Nieder", dass die betreffenden Reste bis und mit maximal 7, vorzugsweise bis und mit maximal 4 Kohlenstoffatome enthalten.

R₂ ist entweder Wasserstoff (dann ist die Verbindung R₂-X der entsprechende Chlor-oder Bromwasserstoff), oder X-SO (dann ist die Verbindung R₂-X das entsprechende Thionylchlorid SOCl₂ oder Thionylbromid SOBr₂). Vorzugsweise ist X Chlor und die Verbindung der Formel R₂-X Chlorwasserstoff oder Thionylchlorid. Auch die Kombination einer Reaktion zunächst mit (beispielsweise wenig) R₂-X, worin R₂ für Wasserstoff steht und X für Chlor oder Brom steht, und anschliessend mit einer Verbindung der Formel R₂-X, worin R₂ X-SO bedeutet, wobei X für Chlor oder Brom steht, ist möglich und kann zu verbesserter Ausbeute führen.

Die Verbindungen der Formel I sind wertvolle Zwischenprodukte oder Vorstufen von Schutzgruppen in der organischen Synthese, insbesondere für die Herstellung von Arzneimitteln und/oder Fungiziden. Sie reagieren mit Aminen, Alkoholen oder Carbonsäuren unter Einführung der Gruppe 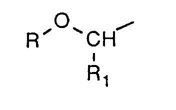 zu den entsprechenden substituierten Aminen, Ethern und Estern. Die Verbindungen der Formel I sind insbesondere geeignet für die Herstellung entsprechender N-Niederalkoxy-alk-1-ylderivate, z.B. der N-[1-(1,3-Dihydroxy-2-niederalkoxy)-niederalk-1-yl]-derivate von Guaninanalogen, beispielsweise den in EP 0 085 424 (publiziert am 10. Aug. 1983), EP 0 187 297 (publiziert am 12. Juli 1986) und in EP 0 532 878 (publiziert am 24. März 1993) beschriebenen Verbindungen, welche antiviral wirksam sind. Beispielsweise kann man 1,3-Dichlor-2-chlormethoxypropan mit einem Alkalimetallsalz, z.B. dem Natriumsalz, von Propionsäure zu 1,3-Dipropionyloxy-2-propionyloxypropan (Beispiel 2 in EP 0 187 297) umsetzen, welches dann gemäss Reaktionsschema II in EP 0 187 297 durch Reaktion mit einem geschützten Guaninderivat und anschliessende Schutzgruppenabspaltung zu 9-(1,3-Dihydroxy-2-propoxymethyl)guanin (= Ganciclovir) umgesetzt werden kann. Analog ist beispielweise die Herstellung von 9-(2-Hydroxy-eth-1-oxymethyl)-guanin (Acyclovir) möglich. Bestimmte Verbindungen der Formel I sind auch geeignet zur Einführung von Schutzgruppen im Laufe von Synthesen, z.B. als Hydroxyschutzgruppen. So kann 2-Methoxyethoxymethylchlorid (1-Chloromethoxy-2-methoxyethan) zur Einführung der 2-Methoxy-ethoxy-methyl-Schutzgruppe (MEM-Gruppe; siehe Corey, E.J., et al., Tetrahedron Lett. 11, 809-812 (1976)) verwendet werden. Diese Schutzgruppe findet beispielsweise bei der Herstellung von 1,6,7,8,9,11a,12,13,14,14a-Decahydro-1,13-di-hydroxy-6-methyl-4H-cyclopent[f]oxacyclotridecin-4-on (Brefeldin A, u. a. antifungischer Stoff) Verwendung (vgl. M. Fieser und L. Fieser, Reagents for Organic Synthesis, Vol 7, S. 228-229, J. Wiley & Sons, New York/Chichester/Brisbane/Toronto 1979).

Im Einzelnen nimmt die Reaktion den folgenden Verlauf, wobei, je nach eingesetztem Reagens der Formel R₂-X, auch folgende erwünschte Nebenprodukte entstehen können:

Ist R₂ gleich Wasserstoff, so ist die Verbindung der Formel R₂-X ein Chlor-oder Bromwasserstoff der Formel HX. Die Reaktion verläuft im wesentlichen wie folgt:

### Reaktionsschema I:

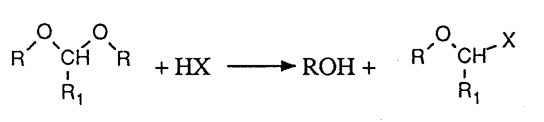

wobei die Reste jeweils die bei der Definition von Verbindungen der Formel I, II und R₂-X genannten Bedeutungen haben. Die entstandene Verbindung der Formel ROH kann beispielsweise verwendet werden, um erneut die Ausgangsverbindung der Formel II herzustellen (s.u.), so dass insgesamt hohe Ausbeuten an Verbindungen der Formel I möglich sind.

Die Verbindung der Formel I wird in überraschend hoher Reinheit gefunden; Unerwünschte Nebenprodukte entstehen nur in unerwartet geringem Umfang, insbesondere finden sich sehr stark verringerte Mengen an den entsprechenden toxischen Bis-(1-halogen-niederalk-1-yl)ethern, wie beispielsweise Bis(chlormethyl)ether - so ist im erfindungsgemässen Ausführungsbeispiel 1 die Menge an Bis(chlormethyl)ether mehr als 200 mal geringer als bei Verfahren aus dem Stand der Technik.

Ausdrücklich ist hervorzuheben, dass N,N-Diniederalkyl-niederalkanoylamide, wie N,N-Dimethylformamid oder N,N-Dimethylacetamid, nicht bei der Reaktion gemäss Reaktionsschema I vorliegen soll.

Ist R₂ gleich X-SO, so ist die Verbindung der Formel R₂-X Thionylchlorid oder ferner Thionylbromid. Die Reaktion verläuft im wesentlichen wie folgt:

### Reaktionsschema II:

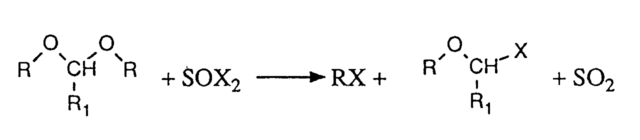

wobei die Reste jeweils die bei der Definition von Verbindungen der Formel I, II und R₂-X genannten Bedeutungen haben. Die Reste RX sind dabei bromierte oder chlorierte Verbindungen, die ihrerseits nützliche Nebenprodukte darstellen (z.B. bei der Herstellung von Agroprodukten, wie Herbiziden (siehe DE-OS 33 03 388, publiziert 11. Aug. 1983, oder US 5,026,896, publiziert am 25. Juni 1991), photographischer Bleichmaterialien (siehe DE-Offen. 26 51 969, publiziert 18. Mai 1977), von Farbstoffen (siehe EP 0 206 114, publiziert am 30. Dez. 1986) oder Pharmazeutika (z.B. Antihypotensiva etc., siehe EP 0 111 455, publiziert am 20. Juni 1984), und so zur Attraktivität der Reaktion beitragen - dies gilt insbesondere für Cl-CH₂-CH₂-O-CH₃.

Auch hier wird die Verbindung der Formel I in überraschend hoher Reinheit gefunden. Unerwünschte Nebenprodukte entstehen nur in unerwartet geringem Umfang, insbesondere finden sich nur ausserordentlich geringe Mengen an den entsprechenden toxischen Bis-(1-halogen-niederalk-1-yl)ethern, wie beispielsweise Bis(chlormethyl)ether - so ist im erfindungsgemässen Ausführungsbeispiel 2 die Menge an Bis(chlormethyl)ether mehr als 100 mal geringer als bei Verfahren aus dem Stand der Technik.

Daneben ist eine unerwartet einfache Aufarbeitung, beispielsweise mittels Destillation, möglich, um die Endprodukte der Formel I in reiner Form zu erhalten.

Um die genannten Vorteile bei der Reaktion mit SOX₂ zu erzielen, ist es erforderlich, dass im Reaktionsgemisch katalytisch wirksame Mengen von einem oder mehreren N,N-Diniederalkyl-niederalkanoylamiden, wie insbesondere N,N-Dimethylformamid oder N,N-Dimethylacetamid, vorliegen, vorzugsweise im gegenüber der Verbindung der Formel II etwa 0,0001- bis 0,1-fachen, vorzugsweise dem 0,001- bis 0,05-fachen molaren Verhältnis. Auch kann das genannte N,N-Diniederalkyl-niederalkanoylamid als Lösungsmittel verwendet werden.

Somit zeigt sich, dass die Verwendung von Acetalen der Formel II zur Herstellung von α-Chlor- und α-Brom-alkylethern der Formel I bei der Umsetzung mit Verbindungen der Formel R₂-X überraschend günstige Resultate hervorruft.

Die Reaktion, worin R₂-X Chlor- oder ferner Bromwasserstoff bedeutet, oder die Reaktion, worin R₂-X Thionylbromid oder insbesondere -chlorid bedeutet, ist jeweils besonders bevorzugt. Im letzteren Fall kann es auch vorteilhaft sein, zunächst eine kleine Menge R₂-X, worin R₂ Wasserstoff bedeutet und X Chlor oder Brom bedeutet, einzusetzen und erst dann die Reaktion mit Thionylbromid oder -chlorid durchzuführen.

Im vor- und nachstehenden Text haben die Reste R, R₁, X und R₂ die vorstehend für Verbindungen der Formel I, II und R₂-X genannten Bedeutungen, sofern nichts anderes angegeben ist.

Die erfindungsgemässen Umsetzungen verlaufen vorzugsweise unter Verwendung der mindestens etwa äquimolaren Menge der Verbindung der Formel R₂-X (bezogen auf die molare Menge der Verbindung der Formel II), in An- oder vorzugsweise Abwesenheit von Lösungs- oder Verdünnungsmitteln, vorteilhaft bei nur wenig erniedrigter bis erhöhter Temperatur und mit anschliessender üblicher Aufarbeitung des Reaktionsgemisches.

Die Umsetzung kann auch in Gegenwart einer Lewissäure als Katalysator durchgeführt werden, sofern R₂-X für Chlor- oder Bromwasserstoff steht; bevorzugt ist die Reaktion ohne Lewissäure. Als Lewissäure können katalytische Mengen, beispielsweise die etwa 0,01- bis etwa 0,1-fache, wie etwa die 0,02 bis 0,05-fache molare Menge (bezogen auf die molare Menge der Verbindung der Formel II) eines Halogenides eines Metalls der Gruppen IIb, IIIb oder IVb des Periodischen Systems der Elemente, wie entsprechende Zink-, Zinn-, Zirkon- und Aluminiumhalogenide, verwendet werden.

Vorteilhaft wird ein geringer, z.B. 1,01- bis 10-facher, vorzugsweise 1,01-bis 8-facher molarer Überschuss der Verbindung der Formel R₂-X gegenüber der Verbindung der Formel II eingesetzt.

Als Lösungsmittel kommen inerte Lösungsmittel in Frage, wie Aromaten, die unter den gegebenen Reaktionsbedingungen jedoch nur dann eingesetzt werden dürfen, wenn sie nicht reagieren, z.B. Benzol oder Toluol, oder Halogenaromaten oder Halogenalkane, z.B. Di-, Tri- oder Tetrachlor-C₁-C₄-alkane, wie Methylenchlorid oder Trichlorethan, oder Chlorbenzol. Vorteilhaft kann die erfindungsgemässe Umsetzung jedoch ohne Lösungsmittel (in Lösung oder in einer Schmelze) vorgenommen werden, wobei die Halogenwasserstoffsäure der Formel R₂-X in gasförmigem Zustand eingeleitet werden kann.

Die Umsetzung wird vorteilhaft im Bereich von wenig erniedrigter bis zu erhöhter Temperatur vorgenommen, vorzugsweise im Temperaturbereich von 0 °C bis 150 °C, beispielsweise 10 bis etwa 120 °C, insbesondere von etwa 15 bis etwa 115 °C, je nach Rückflusstemperatur, welche die obere Grenze bestimmt, sofern nicht unter erhöhtem Druck gearbeitet wird.

Die Reaktion kann ungefähr unter Atmosphärendruck oder bei erhöhtem Druck stattfinden. Der Druck kann vorzugsweise von etwa 0,5 bis zu 250 bar betragen, insbesondere bis zu 50 bar, z.B. bis zu 10 bar.

Die übliche Aufarbeitung erfolgt vorzugsweise auf destillativem Wege, vorzugsweise bei vermindertem Druck, z.B. bei etwa 0,1 bis 200 mbar, wie bei etwa 10 bis etwa 30 mbar. Bei der Reaktion gemäss Reaktionsschema I ist auch die anschliessende Behandlung des gebildeten Alkohols ROH mit Thionylchlorid zum entsprechenden Chlorderivat RCl möglich, welches als solches abdestilliert - hierdurch ist eine Rückreaktion zum Acetal nicht möglich und dadurch die Umsetzung vollständiger. Im Prinzip ist diese Variante eine Kombination zwischen der Reaktion der Reaktionsschemata I und II.

In einer bevorzugten Ausführungsform des erfindungsgemässen Verfahrens erwärmt man ein Gemisch eines Acetals der Formel II auf etwa 50 °C bis 70 °C, leitet über etwa 2 bis 10, insbesondere 4 bis 6, Stunden einen etwa 1,05 bis 8-fachen Überschuss des entsprechenden Chlor- oder Bromwasserstoffs (R₂-X) in Gasform ein; oder tropft während 1 bis 5 Stunden einen 1,01- bis 3-fachen Überschuss an Thionylchlorid oder Thionylbromid (als R₂-X) zu bei Temperaturen zwischen 10 und 115 °C und lässt dann noch 0 bis 5 Stunden weiter unter Rühren reagieren; und trennt das erhaltene Reaktionsgemisch bei vermindertem Druck, vorzugsweise bei etwa 10 mbar bis etwa 30 mbar, destillativ auf.

Die Erfindung betrifft die Herstellung von Verbindungen der Formel I, worin R ein-oder zweifach substituiertes Nieder-, insbesondere C₂-C₄-Alkyl, wie Ethyl, n-Propyl, Isopropyl, n-Butyl, Isobutyl oder sec-Butyl, bedeutet, worin die Substituenten aus Halogen, wie Chlor oder Brom, und Niederalkoxy, wie Methoxy oder Ethoxy, ausgewählt sind, mit der Massgabe, dass diese Substituenten nicht am die Gruppe R an den Rest des Moleküls der Formel I bindenden Kohlenstoffatom des Niederalkylrestes R vorliegen; und worin R₁ Wasserstoff, Niederalkyl, Phenyl oder Phenylniederalkyl bedeutet, insbesondere Wasserstoff oder Nieder-, wie C₁-C₄-Alkyl; und worin X Chlor oder Brom bedeutet, insbesondere Chlor.

Bevorzugt ist die Herstellung von Verbindungen der Formel I, worin R einfach oder zweifach durch Halogen, wie Chlor oder Brom, oder durch C₁-C₄-Alkoxy, wie Methoxy, substituiertes Niederalkyl mit mehr als 2 Kohlenstoffatomen, wie C₂-C₄-Alkyl, bedeutet mit der Massgabe, dass die genannten Substituenten nicht am die Gruppe R an den Rest des Moleküls der Formel I bindenden Kohlenstoffatom des Niederalkylrestes R vorliegen, insbesondere 1,3-Dihalogen-2-C₃-C₇-alkyl, vor allem 1,3-Dihalogen-2-C₃-C₄-alkyl, wie 1,3-Dichlor- oder 1,3-Dibrom-2-propyl, oder 2-(C₁-C₄-Alkoxy)-ethyl; worin R₁ Wasserstoff bedeutet; und worin X Chlor oder Brom, insbesondere Chlor, bedeutet; dadurch gekennzeichnet, dass man die entsprechend substituierten Ausgangsmaterialien verwendet.

Ganz besonders bevorzugt ist die Herstellung der in den Ausführungsbeispielen genannten Verbindungen der Formel I unter den vorstehend definierten allgemeinen oder insbesondere den in den Ausführungsbeispielen genannten Reaktionsbedingungen analogen Verfahrensbedingungen.

Die Ausgangsverbindungen der Formel II sind bekannt oder werden nach an sich bekannten Verfahren hergestellt, oder sie sind kommerziell erhältlich.

Die Herstellung der Acetale der Formel II erfolgt, indem man die entsprechenden Alkohole der Formel III,

R-OH (III)

worin R die genannten Bedeutungen hat, mit entsprechenden Aldehyden der Formel IV,

R₁-CHO (IV)

worin R₁ die genannten Bedeutungen hat, umsetzt unter an sich bekannten Verfahrensbedingungen, beispielsweise den in "Houben-Weyl - Methoden der organischen Chemie", E. Müller et al. (Herausgeber), Band 7, Teil 1, 4. Auflage, Georg Thieme Verlag, Stuttgart auf S. 418 ff. erwähnten Verfahren, oder analog dem in US 2,527,376 (publiziert am 24. Oct. 1950) genannten Verfahren. Die Verbindungen der Formel IV können auch als reaktionsfähige Vorstufen, wie Paraformaldehyd (R₁ = Wasserstoff), eingesetzt werden.

Die direkte Umsetzung der Aldehyde der Formel IV mit den Alkoholen der Formel III führt bei Anwesenheit von Wasserstoffionen über die Stufe des Halbacetals zum Acetal der Formel II; vorzugsweise wird dabei entstehendes Reaktionswasser aus dem Gleichgewicht entfernt, z.B. destillativ oder ferner durch azeotrope Destillation oder durch Umsetzung mit Verbindungen wie Orthokieselsäureester oder Dimethylsulfit, die das entstehende Reaktionswasser umgehend binden.

Die für die Umsetzung erforderlichen Wasserstoffionen werden vorzugsweise nicht durch Halogenwasserstoffsäuren oder nur in Gegenwart von sehr geringen katalytischen Mengen davon (um die Entstehung toxischer Nebenprodukte zu vermeiden); oder in erster Linie durch Zusatz von Schwefelsäure oder aliphatischen oder insbesondere aromatischen Sulfonsäuren, wie Methansulfonsäure, Ethansulfonsäure, Benzolsulfonsäure, (Niederalkylbenzol)sulfonsäure, wie p-Toluolsulfonsäure, oder Naphthalinsulfonsäure, wobei p-Toluolsulfonsäure besonders bevorzugt ist, freigesetzt, wobei die genannten, in erster Linie in Frage kommenden Säuren in katalytischer Menge, beispielsweise in der gegenüber dem Alkohol der Formel III etwa 0,0001- bis 0,01-fachen, vorzugsweise der 0,001-bis 0,01-fachen molaren Menge eingesetzt werden. Die Umsetzung erfolgt vorzugsweise bei erhöhter Temperatur, beispielsweise bei etwa 30 bis 150 °C, insbesondere bei etwa 60 bis etwa 100 °C, vorzugsweise bei etwa 90 °C.

Der Alkohol der Formel III wird vorzugsweise gegenüber der Verbindung der Formel IV im molaren Überschuss zugesetzt, um eine möglichst vollständige Umsetzung zu dem Acetal der Formel II zu erzielen. Vorzugsweise liegt die jeweilige Verbindung der Formel III im 1,5- bis 10-fachen, insbesondere im 1,8- bis 2,5-fachen Überschuss, vor.

Die Reaktion erfolgt in An- oder vorzugsweise Abwesenheit von Lösungsmitteln, beispielsweise den eingangs für die Umsetzung von Verbindungen der Formel II mit Verbindungen der Formel R₂-X genannten Lösungsmitteln.

Erforderlichenfalls wird während der Reaktion entstehendes Reaktionswasser destillativ (z.B. durch azeotrope Destillation) entfernt; das Reaktionswasser kann jedoch auch erst nach der Reaktion entfernt werden.

Die Aufarbeitung erfolgt vorzugsweise durch Destillation des erhaltenen Reaktionsgemisches, insbesondere unter vermindertem Druck, z.B. bei 0,1 bis 100 mbar, wobei Drucke zwischen 1 und 30 mbar besonders bevorzugt sind.

Besonders bevorzugt ist die Reaktion eines Alkohols der Formel III, wie oben definiert, insbesondere von 1,3-Dichlorpropanol oder 2-Methoxy-ethanol, mit einem Aldehyd der Formel IV (oder im Falle des besonders bevorzugten Formaldehyds einer Vorstufe davon, wie Paraformaldehyd oder Trioxan), wobei der Alkohol der Formel III im 1,8-bis 2,5-fachen molaren Überschuss gegenüber dem Aldehyd der Formel IV eingesetzt wird; bei Temperaturen von etwa 60 bis 120 °C in Gegenwart von einer (bezogen auf den Alkohol der Formel III) etwa 0,001- bis 0,01-fachen molaren Menge von Benzol- oder insbesondere Toluolsulfonsäure als Katalysator; mit anschliessender destillativer Entfernung des Reaktionswassers und nachfolgender destillativer Aufarbeitung bei vermindertem Druck, z.B. zwischen etwa 1 und etwa 30 mbar.

Eine besonders bevorzugte Variante bei der Umsetzung mit einer Verbindung der Formel SOX₂, insbesondere mit Thionylchlorid, besteht in einer Kombination der Reaktion des Alkohols der Formel III und des Aldehyds der Formel IV, lediglich unter destillativer Entfernung des Reaktionswassers und gegebenenfalls des nicht umgesetzten Ausgangsmaterials der Formel III, aber ohne nachfolgende Isolierung des Acetals der Formel II in reiner Form (die durch Destillation des Reaktionsgemisches möglich wäre, wie oben beschrieben), und anschliessend direkte Umsetzung des erhaltenen Reaktionsgemisches mit SOX₂ unter den bereits genannten Bedingungen, insbesondere mit Thionylchlorid (also überraschenderweise auch ohne Abtrennung der verwendeten Säuren, welche die für die Reaktion notwendigen Wasserstoffionen liefern, wie der genannten Sulfonsäuren, z.B. p-Toluolsulfonsäure).

Dies bedeutet, im Detail beschrieben, insbesondere ein Verfahren zur Herstellung einer Verbindung der Formel I, worin R, R₁ und X die oben für Verbindungen der Formel I genannten Bedeutungen haben, dadurch gekennzeichnet, dass man einen Alkohol der Formel III,

R-OH (III)

worin R die für Verbindungen der Formel I genannten Bedeutungen hat, mit einem entsprechenden Aldehyd der Formel IV,

R₁-CHO (IV)

worin R₁ die für Verbindungen der Formel I genannten Bedeutungen hat, oder einem reaktionsfähigen Derivat davon, zu einem Acetal der Formel II, 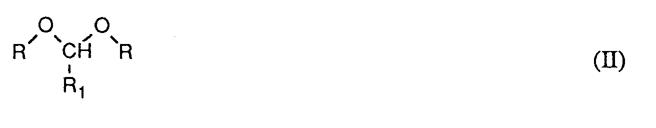 worin R und R₁ die oben genannten Bedeutungen haben, lediglich (vorzugsweise jeweils am Ende der Reaktion) unter destillativer Entfernung des Reaktionswassers und gegebenenfalls des nicht umgesetzten Ausgangsmaterials der Formel III, aber ohne nachfolgende Isolierung des Acetals der Formel II in reiner Form, umsetzt; und anschliessend das erhaltene Reaktionsgemisch direkt weiter mit X₂SO, worin X die für Verbindungen der Formel I genannten Bedeutungen hat, umsetzt, wobei eine katalytisch wirksame Menge an N,N-Diniederalkyl-niederalkanoylamid(en) vorliegen muss.

Diese Variante ist also ein Eintopfverfahren über zwei Stufen; die zusätzlichen Vorteile liegen darin, dass insbesondere bei hochschmelzenden und hochsiedenden Acetalen der Formel II, wie insbesondere dem Bis[(2-chlor-1-chlormethyl)-ethoxy]-methan, die (vor allem im technischen Massstab) relativ aufwendige Reinigung vermieden werden kann und die Synthese ausgehend von den Verbindungen der Formeln III und IV in einem Eintopfverfahren durchgeführt werden kann. Dennoch werden nur sehr geringe Mengen an toxischem Nebenprodukt, wie Bis(chlormethyl)ether, gefunden (vorzugsweise weniger als 20 ppm im Endprodukt der Formel I, das durch Aufarbeitung auf destillativem Wege gewonnen wird, wie oben beschrieben).

Besonders bevorzugt hier ist das Verfahren zur Herstellung von 1,3-Dichlor-2-chlormethoxy-propan der Formel I, welches dadurch gekennzeichnet ist, dass man Formaldehyd oder ein reaktionsfähiges Derivat davon, insbesondere Paraformaldehyd, mit einem molaren Überschuss (vorzugsweise dem 1,5- bis 10-fachen) an 1,3-Dichlorpropan-2-ol in Gegenwart einer aliphatischen oder aromatischen Sulfonsäure, insbesondere der p-Toluolsulfonsäure, in An- oder vorzugsweise Abwesenheit von Lösungsmitteln bei erhöhter Temperatur, insbesondere bei etwa 60 bis 100 °C, umsetzt zu Bis-[(2-chlor-1-chlormethyl)ethoxy]-methan der Formel II, das Reaktionswasser abdestilliert, erforderlichenfalls nicht umgesetztes 1,3-Dichlorpropan-2-ol abdestilliert (vorzugsweise unter vermindertem Druck), und das resultierende Gemisch unter Zufügen eines N,N-Diniederalkyl-niederalkanoylamids (insbesondere des N,N-Dimethylformamids), vorzugsweise im gegenüber dem Bis-[(2-chlor-1-chlormethyl)-ethoxy]-methan der Formel II 0,0001 bis 0,1-fachen molaren Verhältnis, und einer Verbindung der Formel R₂-X, worin R₂ X-SO bedeutet (insbesondere von Thionylchlorid), vorzugsweise im molaren Überschuss gegenüber der Verbindung der Formel II, insbesondere im 1,01- bis 10-fachen Überschuss, wobei die Verbindung der Formel R₂-X vorzugsweise zugetropft wird, bei Temperaturen zwischen 0 und 150 °C, insbesondere zwischen 15 und 115 °C, umsetzt zum Endprodukt, welches durch Aufarbeiten, insbesondere auf destillativem Wege, in reiner Form gewonnen wird. Die einzelnen Verfahrensbedingungen können dabei vorzugsweise den oben jeweils als bevorzugt gekennzeichneten Bedingungen entsprechen.

Wird das Edukt recyclisiert, lassen sich hierbei ohne weiteres Ausbeuten von mehr als 97 % verwirklichen.

Die Erfindung betrifft auch das Verfahren, welches die gesamte Reaktionsabfolge ausgehend von den Alkoholen der Formel III und den Aldehyden über die Acetale der Formel II und deren Umsetzung mit Verbindungen der Formel R₂-X, wie oben definiert, zu den Endprodukten der Formel I einschliesst; im Rahmen dieses Gesamtverfahrens sind die jeweils bei den Einzelschritten als bevorzugt genannten Verfahrensschritte und die den bevorzugten Endprodukten entsprechend substituierten Ausgangsmaterialien ebenfalls besonders bevorzugt.

Die nachfolgenden Beispiele dienen zur Illustration der Erfindung, ohne ihren Umfang in irgendeiner Weise einschränken zu sollen. Temperaturen sind in Grad Celsius (°C) angegeben, Drucke in mbar. Mᵣ bedeutet die relative Molekülmasse. 1,3-Dichlorpropanol steht für 1,3-Dichlorpropan-2-ol.

### Ausführungsbeispiel 1: Herstellung von 1,3-Dichlor-2-chlormethoxy-propan

200 g Bis[(2-chlor-1-chlormethyl)-ethoxy]methan (Mᵣ = 269.9) werden auf etwa 60 °C erwärmt. Unter Rühren werden dann innerhalb von 5 Stunden insgesamt 100 g Chlorwasserstoff (Mᵣ = 36,46) eingeleitet. Nach 2 Stunden wird die Temperatur auf 15 °C herabgesetzt. In einem Aliquot wird die Zusammensetzung des Reaktionsgemisches bestimmt. Die Reaktionsbilanz ist danach die folgende:

| erhaltene Verbindung | Menge |
|---|---|
| Bis-(2-chlor-1-chlormethyl)-ethoxy-methan (Edukt) | 100 g |
| 1,3-Dichlorpropanol (Nebenprodukt) | 47,7 g |
| 1,3-Dichlor-2-chlormethoxy-propan (Produkt) | 65,5 g |
| Bis(chlormethyl)ether (toxisches Nebenprodukt) | 20 mg |

Die Reaktion verläuft im wesentlichen nach folgender Formel:

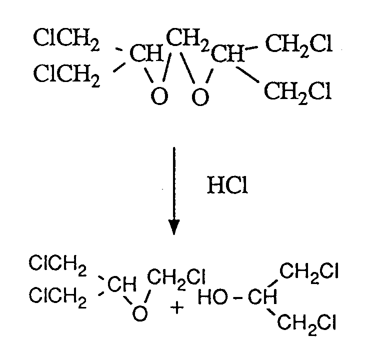

Das Reaktionsgemisch wird unter vermindertem Druck (etwa 20 mbar) destillativ aufgetrennt, man erhält mehr als 99%-ig reines 1,3-Dichlor-2-chlormethoxypropan (Mᵣ = 177,46), welches weniger als 6 ppm des toxischen Bis(chlormethyl)ethers enthält, in einer Ausbeute von 50 %. 1,3-Dichlorpropanol und Bis-(2-chlor-1-chlormethyl)-ethoxy-methan können als Ausgangsstoff wieder eingesetzt werden. Einschliesslich der nachfolgend genannten Vorstufe sind durch diese Möglichkeit der Wiederverwendung Gesamtausbeuten über beide Stufen von über 95 % leicht zu erreichen.

Das Ausgangsmaterial wird wie folgt erhalten:

### Herstellung von Bis-[(2-chlor-1-chlormethyl)-ethoxy]-methan

500 g 1,3-Dichlorpropanol (Mᵣ = 128,9), 64 g Paraformaldehyd (Mᵣ entspricht 30,03 bezogen auf Formaldehyd) und 4,8 g p-Toluolsulfonsäure (Mᵣ = 172,2) werden 2 Stunden bei 90 °C umgesetzt. Anschliessend wird das Reaktionswasser abdestilliert. Das Reaktionsgemisch wird unter vermindertem Druck (etwa 3 mbar) destillativ aufgetrennt, man erhält 374 g etwa 99 % reines Bis-(2-chlor-1-chlormethyl)-ethoxy-methan (Ausbeute: 65 % der Theorie). Die Reaktion verläuft praktisch ohne Bildung von Nebenprodukten. Das noch nicht umgesetzte 1,3-Dichlorpropanol kann erneut in die Reaktion eingesetzt werden, ebenso wie das aus der oben genannten Reaktion zur Herstellung von 1,3-Dichlor-2-chlormethoxy-propan als Nebenprodukt entstandene 1,3-Dichlorpropanol.

Die Reaktion insgesamt (über beide Stufen) ermöglicht somit eine hohe Ausbeute; die abschliessende Reaktion ergibt eine nur äusserst geringe Menge an toxischem Bis(chlormethyl)ether und eine hohe Reinheit des Produktes.

### Vergleichsbeispiel: (analog US 4,568,700):

129 g 1,3-Dichlorpropanol und 30 g Paraformaldehyd werden bei 22 °C unter Rühren innerhalb von 5 Stunden mit 37 g Chlorwasserstoff versetzt. Die Reaktionsbilanz ist wie folgt:

| erhaltene Verbindung | Menge |
|---|---|
| 1,3-Dichlor-2-chlormethoxypropan (Produkt) | 115 g |
| Bis-(2-chlor-1-chlormethyl)-ethoxy-methan | 25,5 g |
| 1,3-Dichlorpropanol (Edukt) | 23 g |
| Bis(chlormethyl)ether (toxisches Nebenprodukt) | 3,2 g |
| unbekannte Nebenprodukte | 10 g |

Das Reaktionsgemisch wird unter vermindertem Druck (etwa 20 mbar) destillativ aufgetrennt. Man erhält nur etwa 97 % reines 1,3-Dichlor-2-chlormethoxy-propan, Ausbeute 56 % der Theorie.

### Ausführungsbeispiel 2: Herstellung von 1-Chlormethoxy-2-methoxy-ethan

200 g Bis(2-methoxyethoxy)-methan (1,218 Mol; Mᵣ = 164,21) und 1 g Dimethylformamid (Mᵣ = 73,10) werden auf etwa 45 °C erwärmt. Unter Rühren werden innerhalb von 2 Stunden 147,8 g Thionylchlorid (1,242 Mol; Mᵣ = 118,97) zugetropft. Man lässt 2 Stunden bei 85 °C nachrühren. Im Verlauf der Umsetzung werden insgesamt 74 g Schwefeldioxid freigesetzt. In einem Aliquot des Reaktionsgemisches wird die Zusammensetzung des Reaktionsgemisches bestimmt. Die Reaktionsbilanz ist folgende:

| erhaltene Verbindung | Menge |
|---|---|
| 1-Chlormethoxy-2-methoxy-ethan (Produkt; Mᵣ = 124,57) | 144 g |
| 2-Methoxy-ethylchlorid (nützliches Nebenprodukt, Mᵣ = 94,54) | 109,4 g |
| Bis-(2-methoxy-ethoxy)-methan | 10 g |
| Bis(chlormethyl)ether | 127 mg |

Die Reaktion verläuft im wesentlichen nach folgender Formel:

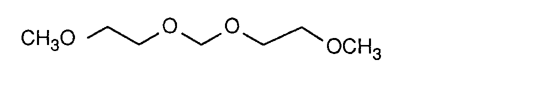

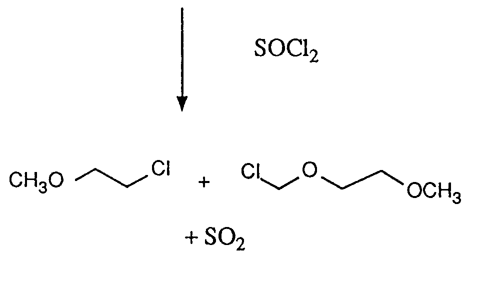

Das Reaktionsgemisch wird unter vermindertem Druck (etwa 20 mbar) destillativ aufgetrennt, man erhält etwa 99 %-ig reines 1-Chlormethoxy-2-methoxy-ethan in einer Ausbeute von 95 % der Theorie.

Das verwendete Dimethylformamid kann nach der Reaktion wiederverwendet werden für denselben Zweck, wobei man vorteilhaft einen Anteil an frischem Dimethylformamid zusetzt, oder für andere Zwecke.

Das Ausgangsmaterial wird wie folgt erhalten:

### Herstellung von Bis(2-methoxyethoxy)methan:

1045 g 2-Methoxy-ethanol (Mᵣ = 76,1), 194 g Paraformaldehyd (Mᵣ = 30,03 bezogen auf Formaldehyd) und 16 g p-Toluolsulfonsäure (Mᵣ = 172,2) werden 2 Stunden bei 90 °C umgesetzt. Anschliessend wird das Reaktionswasser abdestilliert. Das Reaktionsgemisch wird unter vermindertem Druck (etwa 20 mbar) destillativ aufgetrennt. Man erhält 758 g etwa 99 % reines Bis(2-methoxyethoxy)-methan (Mᵣ = 164,21), Ausbeute: 67 % der Theorie. Der Rest (33 %) besteht praktisch vollständig aus dem Edukt, welches recyclisiert werden kann, so dass Gesamtausbeuten von über 97 % resultieren.

Es zeigt sich im Gesamtverfahren eine ausgesprochen niedrige Menge an toxischem Bischlormethylether gebildet wird und ein Produkt von hoher Reinheit entsteht. Nebenbei entsteht das synthetisch wertvolle 2-Methoxy-ethylchlorid.

### Vergleichsbeispiel A: (analog US 4,568,700; vgl. auch E. J. Corey et al., Tetrahedron Lett. 11, 809-812 (1976))

152 g 2-Methoxyethanol und 66 g Paraformaldehyd werden bei 22 °C unter Rühren innerhalb von 2 Stunden mit 72 g Chlorwasserstoff versetzt. In einem Aliquot wird die Zusammensetzung des Reaktionsgemisches bestimmt. Die Reaktionsbilanz ist folgende (unter Ausschluss von Wasser und wasserlöslicher Anteile):

| erhaltene Verbindung | Menge |
|---|---|
| 1-Chlormethoxy-2-methoxy-ethan (Produkt) | 211,7 g |
| Bis(2-methoxyethoxy)-methan | 32,8 g |
| 2-Methoxyethanol | 7,5 g |
| Bis(chlormethyl)ether | 12,6 g |

Das Reaktionsgemisch wird mit 900 ml Pentan verdünnt und über 100 g Magnesiumsulfat getrocknet. Das Gemisch wird unter vermindertem Druck (etwa 20 mbar) destillativ aufgetrennt, man erhält etwa 95 % reines 1-Chlormethoxy-2-methoxy-ethan; Ausbeute 80 % der Theorie.

### Vergleichsbeispiel B (ohne Dimethylformamid):

200 g Bis(2-methoxyethoxy)-methan (1,218 Mol; Mᵣ = 164,21) werden auf etwa 45 °C erwärmt. Unter Rühren werden innerhalb von 2 Stunden 147,8 g Thionylchlorid (1,242 Mol; Mᵣ = 118,97) zugetropft Man lässt 2 Stunden bei 85 °C nachrühren. Im Verlauf der Umsetzung werden insgesamt 13g Schwefeldioxid freigesetzt. In einem Aliquot des Reaktionsgemisches wird die Zusammensetzung des Reaktionsgemisches bestimmt. Die Reaktionsbilanz ist folgende:

| erhaltene Verbindung | Menge |
|---|---|
| 1-Chlormethoxy-2-methoxy-ethan (Produkt; Mᵣ = 124,57) | 121 g |
| 2-Methoxy-ethylchlorid | 17 g |
| Bis-(2-methoxy-ethoxy)-methan | 7 g |
| Verschiedene Nebenprodukte | 180 g |
| Bis(chlormethyl)ether (toxisches Nebenprodukt) | 130 mg |

Das Reaktionsgemisch lässt sich sehr schlecht destillativ auftrennen. Nach Destillation unter vermindertem Druck (etwa 20 mbar) erhält man 165 mg stark verunreinigtes 1-Chlormethoxy-2-methoxy-ethan (64%-ig rein), Ausbeute: 70 %.

### Ausführungsbeispiel 3: Herstellung von 1,3-Dichlor-2-chlormethoxypropan

500 g 1,3-Dichlorpropanol (Mᵣ = 128,9), 64 g Paraformaldehyd (Mᵣ = 30,03) und 4,8 g p-Toluolsulfonsäure (Mᵣ = 172,2) werden 2 Stunden bei 90 °C umgesetzt. Dann wird das Reaktionswasser abdestilliert. Das nicht umgesetzte Ausgangsmaterial 1,3-Dichlorpropanol wird unter vermindertem Druck (etwa 20 mbar) destillativ abgetrennt (kann wiederverwendet werden - ein Rest von etwa 1 oder wenigen Prozent 1,3-Dichlorpropanol im nach der Destillation verbleibenden Reaktionsgemisch kann im Ansatz verbleiben und wirkt sich positiv auf die nachfolgende Reaktion aus). Das Reaktionsgemisch wird auf 60 °C abgekühlt und mit 4,8 g Dimethylformamid (Mᵣ = 73,10) versetzt.

Unter Rühren werden innerhalb von 2 Stunden 177 g Thionylchlorid (Mᵣ = 118,978) zugetropft. Man lässt 3 Stunden bei 110 °C nachrühren. Im Verlauf der Umsetzung werden insgesamt 76 g Schwefeldioxid freigesetzt. In einem Aliqot des Reaktionsgemisches wird die Zusammensetzung bestimmt. Die Reaktionsbilanz ist folgende:

| erhaltene Verbindung | Menge |
|---|---|
| 1,3-Dichlor-2-chlormethoxypropan (Produkt) | 209 g |
| 1,2,3-Trichlorpropan (Nebenprodukt, Mᵣ = 147,43) | 173,8 g |
| Bis[(2-chlor-1-chlormethyl)-ethoxy]-methan (Edukt) | 6,4 g |
| Bis(chlormethyl)ether | 3 mg |

Das Reaktionsgemisch wird unter vermindertem Druck (etwa 10 mbar) destillativ aufgetrennt, man erhält 182 g etwa 99%-ig reines 1,3-Dichlor-2-chlormethoxypropan (Mᵣ = 177,46).

### Vergleichsbeispiel (ohne Dimethylformamid):

500 g 1,3-Dichlorpropanol (Mᵣ = 128,9), 64 g Paraformaldehyd (Mᵣ = 30,03) und 4,8 g p-Toluolsulfonsäure (Mᵣ = 172,2) werden 2 Stunden bei 90 °C umgesetzt. Dann wird das Reaktionswasser abdestilliert. Das nicht umgesetzte Ausgangsmaterial 1,3-Dichlorpropanol wird unter vermindertem Druck (etwa 20 mbar) destillativ abgetrennt. Das Reaktionsgemisch wird auf 60 °C abgekühlt.
Unter Rühren werden innerhalb von 2 Stunden 177 g Thionylchlorid (Mᵣ = 118,978) zugetropft. Man lässt 3 Stunden bei 110 °C nachrühren. Im Verlauf der Umsetzung werden insgesamt 3 g Schwefeldioxid freigesetzt. In einem Aliqot des Reaktionsgemisches wird die Zusammensetzung bestimmt. Die Reaktionsbilanz ist folgende:

| erhaltene Verbindung | Menge |
|---|---|
| 1,3-Dichlor-2-chlormethoxypropan (Produkt) | 187 g |
| Bis[(2-chlor-1-chlormethyl)-ethoxy]-methan (Edukt) | 24 g |
| weitere Nebenprodukte | 240 g |
| Bis(chlormethyl)ether | 3 mg |

Das Reaktionsgemisch lässt sich sehr schlecht destillativ auftrennen. Nach Destillation unter vermindertem Druck (etwa 10 mbar) erhält man stark verunreinigtes 1-Chlormethoxy-2-methoxyethan (76 %-ig rein).

## Patentansprüche

1. Verfahren zur Herstellung von Verbindungen der Formel I, 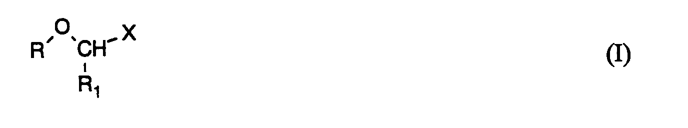 worin
R ein- oder zweifach substituiertes Niederalkyl bedeutet, worin die Substituenten aus Halogen und Niederalkoxy ausgewählt sind, mit der Massgabe, dass diese Substituenten nicht am die Gruppe R an den Rest des Moleküls der Formel I bindenden Kohlenstoffatom des Niederalkylrestes R vorliegen; worin
R₁ Wasserstoff, Niederalkyl, Phenyl oder Phenylniederalkyl bedeutet; und worin
X Chlor oder Brom bedeutet;
dadurch gekennzeichnet, dass man ein Acetal der Formel II, 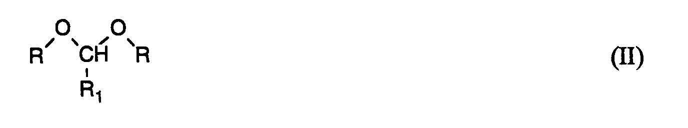 worin R und R₁ die oben genannten Bedeutungen haben,
mit mindestens einer Verbindung der Formel R₂-X, worin R₂ Wasserstoff oder X-SO bedeutet, wobei im letzteren Falle im Reaktionsgemisch noch eine katalytisch wirksame Menge an N,N-Diniederalkyl-niederalkanoylamid(en) vorliegen muss, und worin X die für Verbindungen der Formel I genannten Bedeutungen hat, umsetzt; wobei "nieder" bedeutet, dass die betreffenden Reste bis und mit maximal 7 Kohlenstoffatome enthalten.

2. Verfahren gemäss Anspruch 1 zur Herstellung von Verbindungen der Formel I, worin
R einfach oder zweifach durch Halogen oder durch C₁-C₄-Alkoxy substituiertes Niederalkyl mit mehr als 2 Kohlenstoffatomen bedeutet;
R₁ Wasserstoff bedeutet; und
X Chlor oder Brom bedeutet.

3. Verfahren gemäss Anspruch 1 zur Herstellung von Verbindungen der Formel I, worin
R 1,3-Dihalogen-2-niederalkyl oder 2-(C₁-C₄-Alkoxy)-ethyl bedeutet;
R₁ Wasserstoff bedeutet; und
X Chlor oder Brom, insbesondere Chlor, bedeutet.

4. Verfahren gemäss Anspruch 1 zur Herstellung von 1,3-Dichlor-2-chlormethoxy-propan der Formel I.

5. Verfahren gemäss Anspruch 1 zur Herstellung von 1-Chlormethoxy-2-methoxy-ethan der Formel I.

6. Verfahren gemäss einem der Ansprüche 1 bis 5, dadurch gekennzeichnet, dass man die Reaktion in Abwesenheit von weiteren Lösungsmitteln durchführt.

7. Verfahren gemäss einem der Ansprüche 1 bis 6, dadurch gekennzeichnet, dass man bei der Reaktion einen 1,01- bis 10-fachen, insbesondere 1,01- bis 8-fachen Überschuss der Verbindung der Formel R₂-X gegenüber der Verbindung der Formel II einsetzt.

8. Verfahren gemäss einem der Ansprüche 1 bis 7, dadurch gekennzeichnet, dass man die Reaktion bei Temperaturen zwischen 0 °C und 150 °C, insbesondere zwischen 15 °C und 115 °C, durchführt.

9. Verfahren gemäss einem der Ansprüche 1 bis 8, dadurch gekennzeichnet, dass man die Reaktion bei einem Druck zwischen 0,5 und 250 bar, insbesondere zwischen 0,5 und 10 bar, stattfinden lässt.

10. Verfahren gemäss einem der Ansprüche 1 bis 9, dadurch gekennzeichnet, dass man die anschliessende Aufarbeitung, vorzugsweise bei vermindertem Druck, destillativ durchführt.

11. Verfahren gemäss Anspruch 3, dadurch gekennzeichnet, dass man die Reaktion in Abwesenheit von weiteren Lösungsmitteln durchführt, dass man bei der Reaktion einen 1,01- bis 10-fachen Überschuss der Verbindung der Formel R₂-X gegenüber der Verbindung der Formel II einsetzt, dass man die Reaktion bei Temperaturen zwischen 0 °C und 150 °C durchführt, dass man die Reaktion bei Atmosphärendruck oder bei erhöhtem Druck stattfinden lässt und dass man die anschliessende Aufarbeitung destillativ durchführt.

12. Verfahren gemäss einem der Ansprüche 1 bis 11, dadurch gekennzeichnet, dass man ein Gemisch eines Acetals der Formel II auf 50 °C bis 70 °C erwärmt, über etwa 2 bis 10, insbesondere 4 bis 6, Stunden einen etwa 1,05 bis 8-fachen Überschuss des entsprechenden Chlor- oder Bromwasserstoffs (R₂-X) in Gasform einleitet; oder während 1 bis 5 Stunden einen 1,01- bis 3-fachen Überschuss an Thionylchlorid oder Thionylbromid (als R₂-X) zutropft, wobei man zuvor mit einer geringen Menge an Chlor- oder Bromwasserstoff vorbehandeln kann, bei Temperaturen zwischen 10 und 115 °C, und dann noch 0 bis 5 Stunden weiter unter Rühren reagieren lässt; und das erhaltene Reaktionsgemisch bei vermindertem Druck destillativ auftrennt.

13. Verfahren gemäss Anpruch 1 zur Herstellung einer Verbindung der Formel I, worin R, R₁ und X die in Anspruch 1 genannten Bedeutungen haben, dadurch gekennzeichnet, dass man einen Alkohol der Formel III,
R-OH (III)
worin R die für Verbindungen der Formel I genannten Bedeutungen hat, mit einem entsprechenden Aldehyd der Formel IV,
R₁-CHO (IV)
worin R₁ die für Verbindungen der Formel I genannten Bedeutungen hat, oder einem reaktionsfähigen Derivat davon, zu einem Acetal der Formel II, 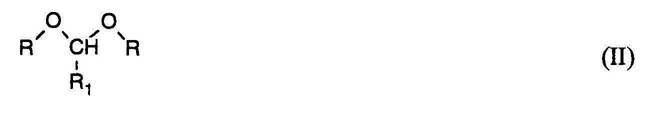 worin R und R₁ die oben genannten Bedeutungen haben, lediglich unter destillativer Entfernung des Reaktionswassers und gegebenenfalls des nicht umgesetzten Ausgangsmaterials der Formel III, aber ohne nachfolgende Isolierung des Acetals der Formel II in reiner Form, umsetzt, und anschliessend das erhaltene Reaktionsgemisch direkt weiter mit X₂SO, worin X die für Verbindungen der Formel I genannten Bedeutungen hat, umsetzt, wobei eine katalytisch wirksame Menge an N,N-Diniederalkyl-niederalkanoylamid(en) vorliegen muss.

14. Verfahren gemäss Anspruch 13 zur Herstellung von 1,3-Dichlor-2-chlormethoxy-propan der Formel I, dadurch gekennzeichnet, dass man Paraformaldehyd mit einem molaren Überschuss von 1,3-Dichlorpropan-2-ol in Gegenwart einer aliphatischen oder aromatischen Sulfonsäure in An- oder Abwesenheit von Lösungsmitteln bei erhöhter Temperatur umsetzt zu Bis-[(2-chlor-1-chlormethyl)-ethoxy]-methan der Formel II, das Reaktionswasser abdestilliert, erforderlichenfalls unter vermindertem Druck nicht umgesetztes 1,3-Dichlorpropan-2-ol abdestilliert; und das Reaktionsgemisch unter Zufügen eines N,N-Diniederalkyl-niederalkanoylamids und einer Verbindung der Formel R₂-X, worin R₂ X-SO bedeutet und X die in Anspruch 1 genannten Bedeutungen hat, bei Temperaturen zwischen 0 und 150 °C umsetzt unter Erhalt des Endproduktes, welches durch Aufarbeiten in reiner Form gewonnen wird.

15. Verfahren gemäss Anspruch 14 zur Herstellung von 1,3-Dichlor-2-chlormethoxy-propan der Formel I, dadurch gekennzeichnet, dass man Paraformaldehyd mit einem 1,5-bis 10-fachen molaren Überschuss von 1,3-Dichlorpropan-2-ol in Gegenwart von p-Toluolsulfonsäure bei etwa 60 bis 100 °C umsetzt zu Bis-[(2-chlor-1-chlormethyl)-ethoxy]-methan der Formel II, das Reaktionswasser abdestilliert, erforderlichenfalls unter vermindertem Druck nicht umgesetztes 1,3-Dichlorpropan-2-ol unter vermindertem Druck abdestilliert; und dem Reaktionsgemisch unter Zufügen von N,N-Dimethylformamid im gegenüber dem Bis-[(2-chlor-1-chlormethyl)-ethoxy]-methan der Formel II 0,0001 bis 0,1-fachen molaren Verhältnis Thionylchlorid im 1,01- bis 10-fachen molaren Überschuss gegenüber der Verbindung der Formel II zutropft, und bei Temperaturen zwischen 15 und 115 °C umsetzt unter Erhalt des Endproduktes, welches auf destillativem Wege in reiner Form gewonnen wird.

## Claims

1. A process for the preparation of a compound of formula I 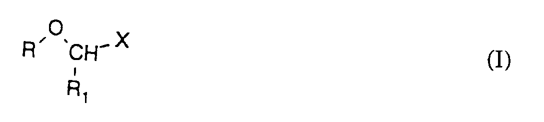 wherein
R is mono- or di-substituted lower alkyl, the substituents being selected from halogen and lower alkoxy, with the proviso that the said substituents are not present at the carbon atom of the lower alkyl radical R linking the group R to the remainder of the molecule of formula I;
R₁ is hydrogen, lower alkyl, phenyl or phenyl-lower alkyl; and
X is chlorine or bromine;
which comprises reacting an acetal of formula II 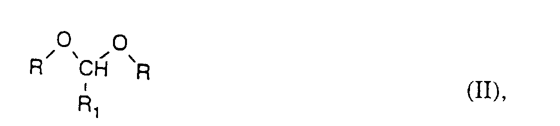 wherein R and R₁ are as defined above,
with at least one compound of formula R₂-X wherein R₂ is hydrogen or X-SO, in which last-mentioned case the reaction mixture must in addition comprise a catalytically effective amount of N,N-di-lower alkyl-lower alkanoylamide(s), and wherein X is as defined for a compound of formula I, "lower" denoting that the radicals in question contain up to and including a maximum of 7 carbon atoms.

2. A process according to claim 1 for the preparation of a compound of formula I, wherein
R is lower alkyl having more than 2 carbon atoms which is mono- or di-substituted by halogen or by C₁-C₄alkoxy;
R₁ is hydrogen; and
X is chlorine or bromine.

3. A process according to claim 1 for the preparation of a compound of formula I, wherein
R is 1,3-dihalo-2-lower alkyl or 2-(C₁-C₄alkoxy)ethyl;
R₁ is hydrogen; and
X is chlorine or bromine, especially chlorine.

4. A process according to claim 1 for the preparation of 1,3-dichloro-2-chloromethoxy-propane of formula I.

5. A process according to claim 1 for the preparation of 1-chloromethoxy-2-methoxyethane of formula I.

6. A process according to any one of claims 1 to 5, which comprises carrying out the reaction in the absence of further solvents.

7. A process according to any one of claims 1 to 6, which comprises using in the reaction a 1.01- to 10-fold excess, especially a 1.01- to 8-fold excess, of the compound of formula R₂-X in relation to the compound of formula II.

8. A process according to any one of claims 1 to 7, which comprises carrying out the reaction in the temperature range from 0°C to 150°C, especially from 15°C to 115°C.

9. A process according to any one of claims 1 to 8, wherein the reaction is carried out at a pressure in the range from 0.5 to 250 bar, especially from 0.5 to 10 bar.

10. A process according to any one of claims 1 to 9, wherein the subsequent working up is carried out by distillation, preferably under reduced pressure.

11. A process according to claim 3, which comprises carrying out the reaction in the absence of further solvents, using in the reaction a 1.01- to 10-fold excess of the compound of formula R₂-X in relation to the compound of formula II, carrying out the reaction in the temperature range from 0°C to 150°C, carrying out the reaction under atmospheric pressure or elevated pressure, and carrying out the subsequent working up by distillation.

12. A process according to any one of claims 1 to 11, which comprises heating a mixture of an acetal of formula II to from 50°C to 70°C, introducing an approximately 1.05- to 8-fold excess of the appropriate hydrogen chloride or hydrogen bromide (R₂-X) in gaseous form over about 2 to 10, preferably 4 to 6, hours; or adding dropwise a 1.01- to 3-fold excess of thionyl chloride or thionyl bromide (as R₂-X) over 1 to 5 hours, prior to which pretreatment can be carried out with a small amount of hydrogen chloride or hydrogen bromide, at a temperature in the range from 10 to 115°C, and then allowing the reaction to proceed, with stirring, for a further 0 to 5 hours; and separating the resultant reaction mixture by distillation under reduced pressure.

13. A process according to claim 1 for the preparation of a compound of formula I, wherein R, R₁ and X are as defined in claim 1, which comprises reacting an alcohol of formula III
R-OH (III)
wherein R is as defined for a compound of formula I, with an appropriate aldehyde of formula IV
R₁-CHO (IV)
wherein R₁ is as defined for a compound of formula I, or with a reactive derivative thereof, to form an acetal of formula II 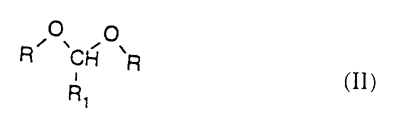 wherein R and R₁ have the above meanings, solely with removal by distillation of the water of reaction and, if necessary, of any unreacted starting material of formula III, but without subsequent isolation of the acetal of formula II in pure form, and thereafter further reacting the resultant reaction mixture directly with X₂SO, wherein X is as defined for a compound of formula I, with the proviso that a catalytically effective amount of N,N-di-lower alkyl-lower alkanoylamide(s) must be present.

14. A process according to claim 13 for the preparation of 1,3-dichloro-2-chloromethoxy-propane of formula I, which comprises reacting paraformaldehyde with a molar excess of 1,3-dichloropropan-2-ol in the presence of an aliphatic or aromatic sulfonic acid in the presence or in the absence of solvents, at elevated temperature, to form bis[(2-chloro-1-chloromethyl)ethoxy]methane of formula II, removing the water of reaction by distillation, if necessary distilling off unreacted 1,3-dichloropropan-2-ol under reduced pressure; and reacting the reaction mixture, with the addition of a N,N-di-lower alkyl-lower alkanoyl-amide and of a compound of formula R₂-X wherein R₂ is X-SO and X is as defined in claim 1, in the temperature range from 0 to 150°C to give the final product, which is obtained in pure form by working up.

15. A process according to claim 14 for the preparation of 1,3-dichloro-1-chloromethoxy-propane of formula I, which comprises reacting paraformaldehyde with a 1.5- to 10-fold molar excess of 1,3-dichloropropan-2-ol in the presence of p-toluenesulfonic acid, in the temperature range of approximately from 60 to 100°C, to form bis[(2-chloro-1-chloromethyl)ethoxy]methane of formula II, removing the water of reaction by distillation, if necessary distilling off unreacted 1,3-dichloropropan-2-ol under reduced pressure; and adding dropwise to the reaction mixture, with the addition of N,N-dimethylformamide in a 0.0001- to 0.1-fold molar ratio in relation to the bis[(2-chloro-1-chloromethyl)ethoxy]methane of formula II, thionyl chloride in a 1.01- to 10-fold molar excess in relation to the compound of formula II, and reacting in the temperature range from 15 to 115°C to give the final product, which is obtained in pure form by distillation.

## Revendications

1. Un procédé de préparation des composés de formule I 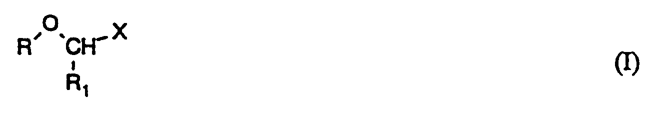 où
R signifie un groupe alkyle inférieur mono- ou disubstitué, où les substituants sont choisis parmi un halogène et un groupe alcoxy inférieur, avec la condition que ces substituants ne soient pas présents sur l'atome de carbone du reste alkyle inférieur R reliant le groupe R au reste de la molécule de formule I; où
R₁ signifie l'hydrogène, un groupe alkyle inférieur, phényle ou phényl-alkyle inférieur; et où
X signifie le chlore ou le brome;
caractérisé en ce qu'on fait réagir un acétal de formule II 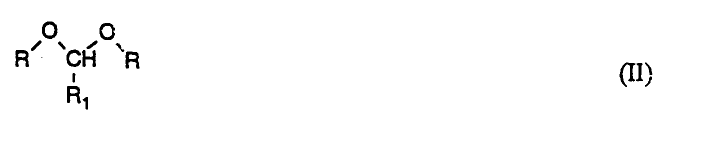 où R et R₁ ont les significations indiquées plus haut,
avec au moins un composé de formule R₂-X, où R₂ signifie l'hydrogène ou X-SO, où dans le dernier cas, dans le mélange réactionnel on doit encore trouver une quantité efficace du point de vue catalytique de N,N-di-alkyl inférieur-alcanoylamide(s) inférieur, et où X a les significations indiquées pour les composés de formule I; "inférieur" signifiant dans ce cas là, que les restes concernés contiennent jusqu'à et au maximum 7 atomes de carbone.

2. Un procédé selon la revendication 1, pour la préparation des composés de formule I, où R signifie un groupe alkyle inférieur ayant plus de 2 atomes de carbone mono- ou disubstitué par un halogène ou un groupe C₁-C₄-alcoxy;
R₁ signifie l'hydrogène; et
X signifie le chlore ou le brome.

3. Un procédé selon la revendication 1, pour la préparation des composés de formule I, où
R signifie un groupe 1,3-dihalogène-2-alkyle inférieur ou un groupe 2-(C₁-C₄-alcoxy)-éthyle;
R₁ signifie l'hydrogène; et
X signifie le chlore ou le brome, en particulier le chlore.

4. Un procédé selon la revendication 1, pour la préparation du 1,3-dichloro-2-chlorométhoxy-propane de formule I.

5. Un procédé selon la revendication 1, pour la préparation du 1-chlorométhoxy-2-méthoxy-éthane de formule I.

6. Un procédé selon l'une des revendications 1 à 5, caractérisé en ce qu'on effectue la réaction en l'absence d'autres solvants.

7. Un procédé selon l'une des revendications 1 à 6, caractérisé en ce que pour la réaction, on utilise un excès de 1,01 à 10 fois, en particulier de 1,01 à 8 fois du composé de formule R₂-X par rapport au composé de formule II.

8. Un procédé selon l'une des revendications 1 à 7, caractérisé en ce qu'on effectue la réaction à des températures comprises entre 0°C et 150°C, en particulier entre 15°C et 115°C.

9. Un procédé selon l'une des revendications 1 à 8, caractérisé en ce que la réaction s'effectue sous une pression comprise entre 0,5 et 250 bar, en particulier entre 0,5 et 10 bar.

10. Un procédé selon l'une des revendications 1 à 9, caractérisé en ce qu'on effectue le traitement final par distillation, de préférence sous une pression réduite.

11. Un procédé selon la revendication 3, caractérisé en ce qu'on effectue la réaction en l'absence d'autres solvants, en ce qu'on utilise lors de la réaction un excès de 1,01 à 10 fois du composé de formule R₂-X par rapport au composé de formule II, en ce qu'on effectue la réaction à des températures comprises entre 0°C et 150°C , en ce que la réaction a lieu sous la pression atmosphérique ou sous une pression supérieure et en ce qu' on effectue le traitement final par distillation.

12. Un procédé selon l'une des revendications 1 à 11, caractérisé en ce qu'on chauffe un mélange d'un acétal de formule II à 50°C jusqu'à 70°C, on introduit un excès de 1,05 à 8 fois d'acide chlorhydrique ou d'acide bromhydrique (R₂-X) correspondant sous forme gazeuse, pendant environ 2 à 10 heures, en particulier entre 4 et 6 heures; ou bien pendant 1 à 5 heures, on ajoute goutte à goutte un excès de 1,01 à 3 fois de chlorure de thionyle ou de bromure de thionyle (sous forme de R₂-X), mais dans ce cas là, un pré-traitement avec une faible quantité d'acide chlorhydrique ou d'acide bromhydrique peut être effectué au préalable à des températures comprises entre 10 et 115°C et on laisse ensuite réagir sous agitation pendant encore 0 à 5 heures; et le mélange réactionnel obtenu est ensuite séparé par distillation sous pression réduite.

13. Un procédé selon la revendication 1, pour la préparation d'un composé de formule I, où R, R₁ et X ont les significations indiquées à la revendication 1, caractérisé en ce qu'on fait réagir un alcool de formule III
R-OH (III)
où R a les significations indiquées pour les composés de formule I, avec un aldéhyde correspondant de formule IV
R₁-CHO (IV)
où R₁ a les significations indiquées pour les composés de formule I, ou avec un de ses dérivés réactifs, en un acétal de formule II 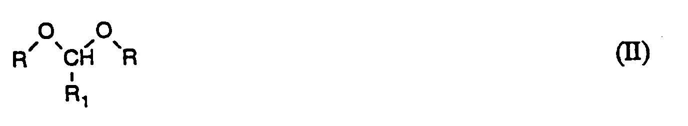 où R et R₁ ont les significations indiquées plus haut, uniquement avec élimination par distillation de l'eau de réaction et éventuellement du produit de départ de formule III qui n'a pas réagi, mais sans isolement ultérieur de l'acétal de formule II sous forme pure, et on fait réagir ensuite le mélange réactionnel obtenu directement avec X₂SO, où X a les significations indiquées pour les composés de formule I, une quantité efficace du point de vue catalytique de N,N-di-alkyl inférieur-alcanoylamide(s) inférieur devant être présente.

14. Un procédé selon la revendication 13, pour la préparation du 1,3-dichloro-2-chlorométhoxypropane de formule I, caractérisé en ce qu'on fait réagir le paraformaldéhyde avec un excès molaire de 1,3-dichloropropane-2-ol en présence d'un acide sulfonique aliphatique ou aromatique en présence ou en l'absence de solvants à une température élevée, en bis-[(2-chloro-1-chlorométhyl)-éthoxy]-méthane de formule II, on élimine l'eau de réaction par distillation, si nécessaire, on élimine par distillation sous une pression réduite le 1,3-dichloropropane-2-ol qui n'a pas réagi; et on fait réagir le mélange réactionnel avec addition de N,N-di-alkyl inférieur-alcanoylamide inférieur et d'un composé de formule R₂-X, où R₂ signifie X-SO et X a les significations indiquées à la revendication 1, à des températures comprises entre 0 et 150°C, pour obtenir le produit final, qui, après traitement, est obtenu sous forme pure.

15. Un procédé selon la revendication 14, pour la préparation du 1,3-dichloro-2-chlorométhoxypropane de formule I, caractérisé en ce qu'on fait réagir le paraformaldéhyde avec un excès molaire de 1,5 à 10 fois de 1,3-dichloropropane-2-ol en présence d'acide p-toluènesulfonique, à une température comprise entre environ 60 à 100°C, en bis-[(2-chloro-1-chlorométhyl)-éthoxy]-méthane de formule II, on élimine l'eau de réaction par distillation, si nécessaire, on élimine par distillation sous une pression réduite le 1,3-dichloropropane-2-ol qui n'a pas réagi; et, au mélange réactionnel, avec addition de N,N-diméthylformamide en un excès molaire de 0,0001 à 0,1 fois par rapport au bis-[(2-chloro-1-chlorométhyl)-éthoxy]-méthane de formule II, on ajoute goutte à goutte du chlorure de thionyle dans un excès molaire de 1,01 à 10 fois par rapport au composé de formule II, et on fait réagir à des températures comprises entre 15 et 115°C, ce qui donne le produit final qui est obtenu par distillation sous forme pure.
